**Europäisches Patentamt**

**European Patent Office**

**Office européen des brevets**

(19)

(11) Veröffentlichungsnummer : **0 494 066 A2**

# (12) EUROPÄISCHE PATENTANMELDUNG

(21) Anmeldenummer : **91890311.3**

(22) Anmeldetag : **24.12.91**

(51) Int. Cl.$^5$ : **G01N 1/10,** G01N 35/06

(30) Priorität : **02.01.91 AT 5/91**

(43) Veröffentlichungstag der Anmeldung :
**08.07.92 Patentblatt 92/28**

(84) Benannte Vertragsstaaten :
**AT CH DE DK ES IT LI NL**

(71) Anmelder : **EBNER ELECTRONIC
GESELLSCHAFT m.b.H.
Neuhofenstrasse 35
A-4810 Gmunden (AT)**

(72) Erfinder : **Ebner, Walter, Ing.
Greimlhofstrasse 13
A-4813 Altmünster (AT)**

(74) Vertreter : **Hübscher, Heiner, Dipl.-Ing. et al
Spittelwiese 7
A-4020 Linz (AT)**

(54) **Probenahmevorrichtung für eine Milchqualitätsprüfung.**

(57) Eine Probenahmevorrichtung für eine Milchqualitätsprüfung weist einen Probenkasten (1) zur Aufnahme mehrerer nebeneinander angeordneter, jeweils eine Reihe von Probenbehältern (3) aufweisender Stative (2), einen Positionsmelder (9) zum Identifizieren der einzelnen Behälter (3) und einer von Behälter zu Behälter relativbewegbaren Abfülleinrichtung (5) zum Einfüllen der Proben auf, wobei jedes Stativ (2) eine bestimmte Stativmarkierung (6) trägt und in Stativlängsrichtung Reihenmarkierungen (7) für die Behälterpositionen innerhalb einer Behälterreihe vorgesehen sind und der Positionsmelder (9) Leseeinrichtungen (10, 11) zum Auslesen dieser Markierungen (6, 7) in Abhängigkeit von der jeweiligen Lage der Abfülleinrichtung (5) umfaßt.

Um die eindeutige, von der Zuordnung der einzelnen Behälter (3) zu ihren jeweiligen Positionen abhängigen Identifizierung der Behälter bzw. Milchproben zu gewährleisten, sind die Stative (2) und die ihnen zugehörigen Probenbehälter (3) jeweils als feste Prüfeinheit (12) ausgebildet.

EP 0 494 066 A2

Die Erfindung bezieht sich auf eine Probenahmevorrichtung für eine Milchqualitätsprüfung, mit einem Probenkasten zur Aufnahme mehrerer nebeneinander angeordneter, jeweils eine Reihe von Probenbehältern aufweisender Stative, einem Positionsmelder zum Identifizieren der einzelnen Behälter und einer von Behälter zu Behälter relativbewegbaren Abfülleinrichtung zum Einfüllen der Proben in die Behälter, wobei jedes Stativ eine bestimmte Stativmarkierung trägt und in Stativlängsrichtung Reihenmarkierungen für die Behälterpositionen innerhalb einer Behälterreihe vorgesehen sind und der Positionsmelder Leseeinrichtungen zum Auslesen dieser Markierungen in Abhängigkeit von der jeweiligen Lage der Abfülleinrichtung umfaßt.

Um die von einem Lieferanten übernommene Milch hinsichtlich Menge und Qualität überprüfen zu können, ist es notwendig, am Übernahmeort nicht nur die in den Sammelwagen übernommene Milchmenge festzustellen, sondern auch aus dieser Übernahmemenge eine Milchprobe zu ziehen und die lieferantenspezifischen Daten denen der jeweiligen Milchmenge und der zugehörigen Milchprobe zuzuordnen. Dazu werden die Proben in entsprechende Probenbehälter gefüllt und die Zuordnung erfolgt über die Identifizierung dieser Behälter. Wie die DE-PS 37 00 654 zeigt, ist es bereits bekannt, die Probenbehälter reihenweise in Stativen und die Stative nebeneinander in entsprechend rechteckigen Probenkasten anzuordnen, so daß die Position des einzelnen Behälters durch seinen Platz in der Behälterreihe und sein bestimmtes Stativ genau fixierbar ist. Werden die einzelnen Stative und die Behälterreihen mit geeigneten Markierungn versehen, können die Behälter positionsabhängig identifiziert werden, in die gerade eine Probe eigefüllt wird. Bei maschinenlesbaren Markierungen und entsprechenden Datenverarbeitungseinrichtungen lassen sich so automatisch von den eingesammelten Milchlieferungen die Mengen, die die jeweiligen Milchproben aufnehmenden Probenbehälter und die zugehörigen Lieferantenangaben erfassen und einander zuordnen.

Um die gewünschte Zuordnungssicherheit zu erreichen, muß dafür gesorgt sein, daß nicht nur der jeweilige Platz des Probenbehälters identifizierbar ist, sondern auch der diesem Platz zugeordnete Probenbehälter tatsächlich durch diese Positionsidentifizierung bestimmt wird. Bei den bekannten Probenahmevorrichtungen sind die Probenbehälter als Einzelflaschen ausgebildet und auch lose in den Stativen eingesetzt. Die beim Abfüllen der Probe im Probekasten eingenommene Behälterposition, die Grundlage für die Probenidentifizierung ist, könnte daher nach der Probennahme durch gewolltes oder ungewolltes Vertauschen der Behälter verlorengehen, was beim Abnehmen des Probenkastens vom Milchsammelwagen und Übergeben an das Milchlabor für die eigentliche Qualitätsprüfung der Milchproben, beim Umsortieren der Probenflaschen vom Probenkasten des Sammelwagens in einen Prüfkasten des Milchlabors usw. durchaus möglich wäre. Die einwandfreie Zuordnung einer Milchprobe zu einem bestimmten Lieferanten ist daher nicht lückenlos und unfehlbar nachzuvollziehen und daher auch dem Vorwurf einer vertauschten Milchprobe nicht wirklich schlüssig zu begegnen, wenn eine rationelle Positionsidentifizierung der Probenbehälter durch ein von der Stativnummer und dem Reihenplatz des jeweiligen Stativs abhängiges Koordinatensystem dem Aufwand einer Einzelmarkierung jedes Probenbehälters vorgezogen wird.

Der Erfindung liegt daher die Aufgabe zugrunde, diese Mangel zu beseitigen und eine Probennahmevorrichtung der eingangs geschilderten Art zu schaffen, die auf einfache Weise im Zusammenhang mit einer Positionsidentifikation auch eine sichere Behälteridentifikation garantiert.

Die Erfindung löst diese Aufgabe dadurch, daß die Stative und die ihnen zugehörenden Probenbehälter jeweils als feste Prüfeinheit ausgebildet sind. Da jedes Stativ mit seinen zugehörenden Probenbehältern als untrennbares Ganzes vorhanden ist, wird jeder Manipulation eines Einzelbehälters vorgebeugt und Stativ und Behälter sind einheitlich zu handhaben. Das bringt nicht nur eine Erleichterung der Hantierbarkeit mit sich, sondern macht auch eine fälschliche Umsortierung der Behälter unmöglich und sorgt für die bleibende Zuordnung von Behälter zu Behälterposition. Da jedes Stativ eine bestimmte Stativmarkierung besitzt, ist damit gleichzeitig auch jede Prüfeinheit an sich identifizierbar, wobei die Platznummer in der Behälterreihe der Prüfeinheit gemeinsam mit der Stativnummer die exakte Behälteridentifizierung gewährleistet. Vorausgesetzt dabei ist, daß die Reihenmarkierungen nicht in verkehrter Richtung ausgelesen werden können, was aber durch eine geeignete Verknüpfung zwischen Stativnummer und Reihenmarkierung einfach zu lösen ist.

Die Prüfeinheiten können durch geeignetes Befestigen der Einzelbehälter in entsprechenden Aufnahmen des Stativs, etwa durch Einkleben zustandekommen, besonders vorteilhaft ist es aber, wenn die aus Stativ und Probenbehältern bestehenden Prüfeinheiten jeweils einstückig, z.B. aus Glas, aus Kunststoff oder Metall, hergestellt sind, was den Herstellungsaufwand senkt und auch eine Vereinfachung der Proben- und Stativform ermöglicht.

Sind die Prüfeinheiten mit einer sich über die gesamte Behälterreihe erstreckenden Deckelleiste verschließbar, läßt sich die Prüfeinheit gemeinsam verschließen und verhindern, daß einzelne Behälter für sich geöffnet werden können, was die Gefahr eines Probenmißbrauches praktisch ausschließt, da damit auch ein Umleeren einzelner Behälter unmöglich wird. Darüber hinaus ist die Handhabung eines gemeinsamen Deckels geschickter als die einer Mehrzahl von für die Einzelbehälter vorgesehenen Einzeldeckeln und es wird auch eine unhandliche gemeinsame Verschlußplatte für einen ganzen Probenkasten unnötig.

Ist die Stativmarkierung auf einem in einem Eckkantenbereich der Prüfeinheit schräg zwischen Stirn- und Längsseite oder auf einem an der Unterseite der Prüfeinheit angeordneten Markierungsträger aufgebracht, kann diese Stativmarkierung sowohl von der Stirnseite als auch von der Längsseite bzw. von der Unterseite her gelesen werden, was die Zugänglichkeit für einen Lesekopf auch beineben- und/oder hintereinandergereihten Prüfeinheiten sicherstellt.

Besonders günstig ist es, wenn die Prüfeinheiten in eine Markierungsschiene einsetzbar sind, die den Reihenmarkierungen für den Probenkasten entsprechende Positionsmarkierungen aufweist. Üblicherweise sind die Reihenmarkierungen für alle Stative gemeinsam auf einer probenkastenfesten Markierungsleiste aufgebracht, von wo sie durch die Leseeinrichtung des Positionsgebers ausgelesen und mit der zugehörigen Stativnummer zur Positionsnummer kombiniert werden. Die einzelnen Stative selbst brauchen daher keine eigenen Reihenmarkierungen mehr aufzuweisen. Um dennoch die Identifizierungssicherheit auch außerhalb des Probenkastens, beispielsweise im Milchlabor, rationell herzustellen, ist für diese Prüfeinheiten eine Markierungsschiene vorgesehen, die ihrerseits die entsprechenden Reihenmarkierungen trägt und nun für jede eingesetzte Prüfeinheit gemeinsam mit der jeweiligen Stativmarkierung die exakte Positionsidentifizierung ermöglicht. Zweckmäßigerweise wird die Prüfeinheit nur in einer bestimmten Richtung in die Markierungsschiene einsetzbar sein, um die Reihenfolge der Platzmarkierungen sicherzustellen, und mit einer solchen Markierungsschiene ist die Identifizierung der Proben von allen Prüfeinheiten im Labor gewährleistet.

Sind nach einer weiteren Ausgestaltung der Erfindung die Prüfeinheiten mit einem der Länge nach verlaufenden, die Reihenmarkierungen tragenden Markierungsstreifen versehen, kommt es auf rationelle Weise zu einer mit einer Einzelmarkierung jedes Behälters vergleichbaren Probenmarkierung. Dabei läßt sich dieser Markierungsstreifen mit wenig Mehraufwand als Codespur mit einem Binär; Sicherheitscode u.dgl. für alle Prüfeinheiten gleichhalten, da ja immer die gleichen Platzpositionen zu bezeichnen sind, so daß mit diesen Prüfeinheiten eine exakte Einzelidentifizierung der Behälter möglich ist und es dazu keiner weiteren Markierungsleisten od.dgl. im Bereich der Probenahmevorrichtung oder der Prüfeinrichtungen bedarf.

Zum Abfüllen der Proben in die Probenbehälter und zum Identifizieren dieser Probenbehälter werden die Abfülleinrichtung und die Leseeinrichtungen des Positionsmelders zweckmäßigerweise auf einem aus Längsschlitten und Querschlitten zusammengesetzten Kreuzschlitten aufgebaut. Dabei ist bisher der Querschlitten mit einem Lesekopf zum Auslesen der Stativmarkierungen versehen und der Längsschlitten trägt einen Lesekopf zum Auslesen der auf einer mitfahrenden Markierungsleiste angebrachten Reihenmarkierungen. Um diesen konstruktiven Aufwand zu vereinfachen, kann erfindungsgemäß die Leseeinrichtung für die Stativmarkierungen einen der Lage der jeweiligen Markierungsträger entsprechend nebeneinander gereihte Einzelsensoren umfassenden Mehrfachlesekopf und einen am Querschlitten sitzenden, einer längsschlittenfesten Markierungsleiste zugeordneten Positionsgeber zur Sensoraktivierung aufweisen und können die Reihenmarkierungen auf einem schlittenunabhängigen Längsträger, beispielsweise eine kastenfeste Markierungslängsleiste aufgebracht und über einen am Längsschlitten sitzenden Lesekopf auslesbar sein. Damit werden die Stativmarkierungen über einen fest angeordneten Mehrfachlesekopf ausgelesen, was nicht nur eine einfachere Bauweise bedingt, sondern auch eine beliebige Anpassung an vorhandene Probenkasten erlaubt, und da auch die Reihenmarkierungen auf einer ortsfesten Markierungslängsleiste aufgebracht sind, wird die Auslesegenauigkeit verbessert und es kommt zu keinen mechanischen Führungsschwierigkeiten mehr.

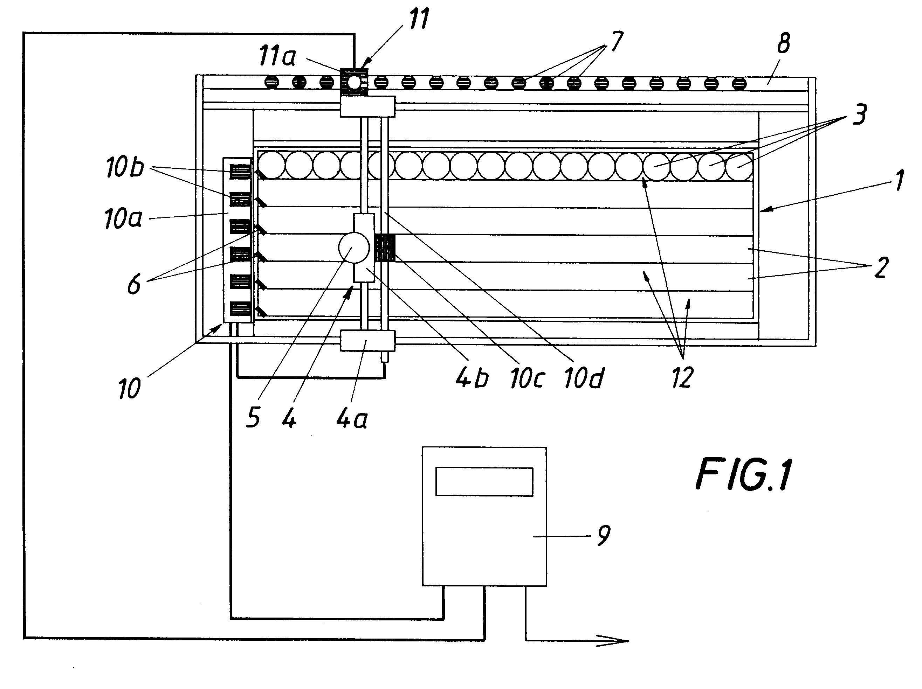

In der Zeichnung ist der Erfindungsgegenstand rein schematisch veranschaulicht, und zwar zeigen
Fig. 1 einen Teil einer erfindungsgemäßen Probenahmevorrichtung in Draufsicht,
Fig. 2 - 4 eine Prüfeinheit für diese Probenahmevorrichtung in Seitenansicht, Stirnansicht und Draufsicht,
Fig. 5 und 6 eine Markierungsschiene für die Prüfeinheiten in Seiten- und Stirnansicht und
Fig. 7 ein weiteres Ausführungsbeispiel einer Prüfeinheit in Seitenansicht.

Eine Probenahmevorrichtung für eine Milchqualitätsprüfung weist einen Probenkasten 1 auf, in dem mehrere Stative 2 mit jeweils einer Reihe von Probenbehälter 3 nebeneinander angeordnet sind. Auf einem Kreuzschlitten 4, der sich aus einem in Längsrichtung der Stative 3 verfahrbaren Längsschlitten 4 a und einem quer dazu verfahrbaren Querschlitten 4 b zusammensetzt, ist eine Abfülleinrichtung 5 von Behälter zu Behälter bewegbar, mit der Milchproben in die jeweiligen Probenbehälter 3 eingefüllt werden können. Zur Identifizierung des jeweiligen Behälters 3 trägt jedes Stativ 2 im Bereich einer Stirnseite eine Stativmarkierung 6 und jeder Platz eines Behälters 3 innerhalb der Behälterreihe ist über Reihenmarkierungen 7 auf einem kastenfesten Längsträger 8 bestimmbar. Zum Erkennen dieser Markierungen 6, 7 gibt es einen Positionsmelder 9, der mit einer Leseeinrichtung 10 für die Stativmarkierung 6 und einer Leseeinrichtung 11 für die Längsmarkierungen zusammenwirkt und diese Positionskenndaten einem nicht weiter dargestellten Datenverarbeitungsgerät eingibt. Die Leseeinrichtung 10 umfaßt einen kastenfest angeordneten Mehrfachlesekopf 10a, der den einzelnen Stativmarkierungen 6 gegenüberliegende Einzelsensoren 10 b aufweist, und einen am Querschlitten 4 b vorgesehenen Positionsgeber 10 c, der mit dem Querschlitten 4 b entlang einer längsschlittenfesten Markierungsleiste 10 d verfahrbar ist und entsprechend der jeweiligen Lage über einem der Stative 2 den zugehörigen Einzelsensor 10 b zum Auslesen der Markierung 6 aktiviert. Die Leseeinrichtung 11 für die Reihenmarkierungen 7 besteht aus einem Lesekopf 11 a, der mit dem Längsschlitten 4 a verbunden ist und dem Längsträger 8 entlang verfährt, so daß das Auslesen der Reihenmarkierungen 7 in Abhängigkeit von der jeweiligen Reihenposition der Abfülleinrichtung 5 erfolgt und zusammen mit der jeweiligen Stativmarkierung eine eindeutige Identifizierung des jeweiligen Behälters 3 möglich ist. Als zu dieser Identifizierung verwendbare Markierungen sind praktisch alle maschinenlesbaren Markierungen und Codierungen geeignet, von Reed-Kontakten bis kontaktlos lesbaren Halbleiterspeicher u.dgl. Es gibt keinerlei Beschränkung hinsichtlich dieser Markierungen.

Bei einer Milchübernahme von einem beliebigen Lieferanten wird in nicht weiter dargestellter Weise von der übernommenen Milch ein Teilstrom als Milchprobe abgezweigt und der Abfülleinrichtung 5 zugeführt, die sie in einen der Probenbehälter 3 des Probenkastens 1 abfüllt. Damit nun diese Probe dem jeweiligen Lieferanten zugeordnet werden kann, wird die Position des Behälters 3, in den die Probe eingefüllt wurde, über die Leseeinrichtungen 10, 11 des Positions melders 9 ausgelesen und über den Positionsmelder 9 dem Datenverarbeitungsgerät eingegeben, in dem diese Position den Daten des Lieferanten selbst und der beim Übernehmen gemessenen Milchmenge zugeordnet wird, womit die Probe in diesem Probenbehälter 3 identifizierbar und als dem entsprechenden Lieferanten zugehörig bestimmbar ist.

Um sicherzustellen, daß das Probengefäß 3 nach der Probennahme nicht ausgetauscht und diese Probe tatsächlich durch die Behälterposition identifiziert werden kann, bildet jedes Stativ 2 mit den zugehörigen Probenbehältern 3 eine Prüfeinheit 12, durch die Behälter 3 und Stativ 2 untrennbar miteinander verbunden sind. Es kommt zu einer leichten Hantierbarkeit dieser Prüfeinheiten, und durch die bleibende Zugehörigkeit der Behälter 3 zum jeweiligen Stativ 2 ist ein fälschliches Umsortieren der einzelnen Probenbehälter ausgeschlossen. Die Prüfeinheiten 12 sind mit einer gemeinsamen, durchgehenden Deckelleiste 13 verschlossen und die Stativmarkierung 6 sitzt jeweils auf einem im Eckkantenbereich, vorzugsweise unter 45° geneigt, zwischen Stirn- und Längsseite angeordneten Markierungsträger 6 a, so daß diese Stativmarkierung gleich gut sowohl von der Längsseite als auch von der Breitseite auslesbar ist. Die Prüfeinheiten 12 werden zur Überprüfung der Milchqualität in ein Milchlabor gebracht, wo die entsprechenden Milchuntersuchungen vorgenommen werden können. Dazu sind dip Prüfeinheiten 12 dem Probenkasten 1 zu entnehmen und den entsprechenden Prüfeinrichtungen zuzuführen. Damit hier die eindeutige Identifizierung der Probenbehälter 3 erhalten bleibt und nicht an der Prüfeinrichtung weitere Markierungsleisten od. dgl. vorgesehen werden müssen, gibt es eine Markierungsschiene 14, in die die Prüfeinheiten 12 einsetzbar sind und die längsseitig den Reihenmarkierungen 7 entsprechende Positionsmarkierungen 7 a aufweisen. Diese Positionsmarkierungen 7 a erlauben zusammen mit der entsprechenden Stativmarkierung 6 auch unabhängig vom Probenkasten 1 die entsprechende Positionserkennung der Probenbehälter 3 und damit die eindeutige Probenidentifizierung in der Prüfeinrichtung, die nur die entsprechenden Leseeinrichtungen aufweisen muß.

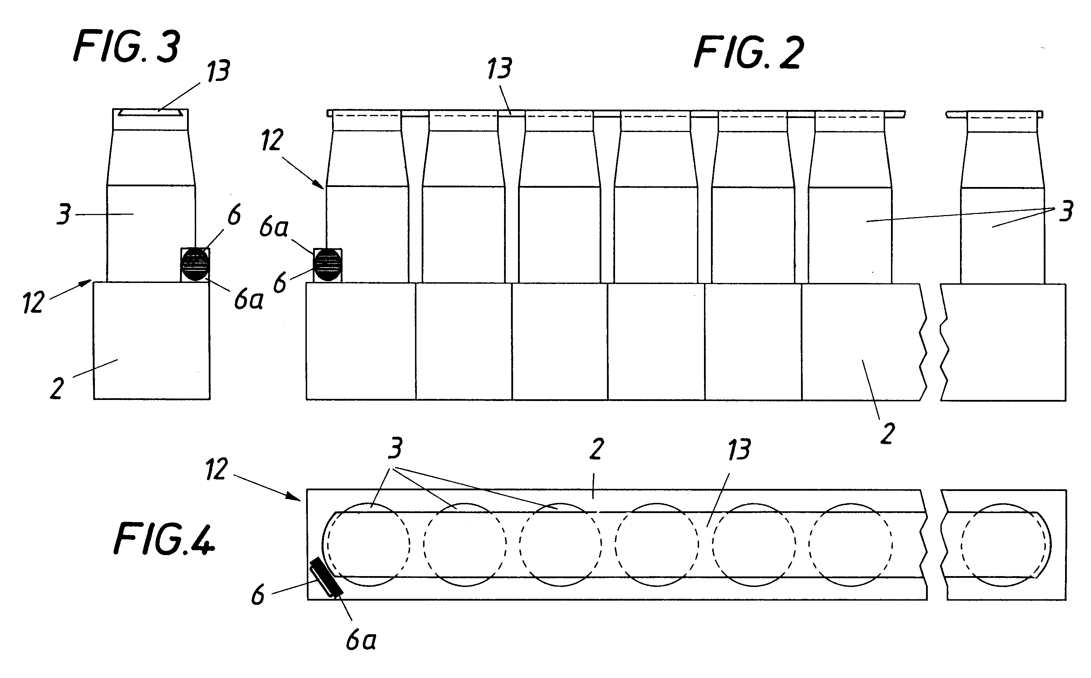

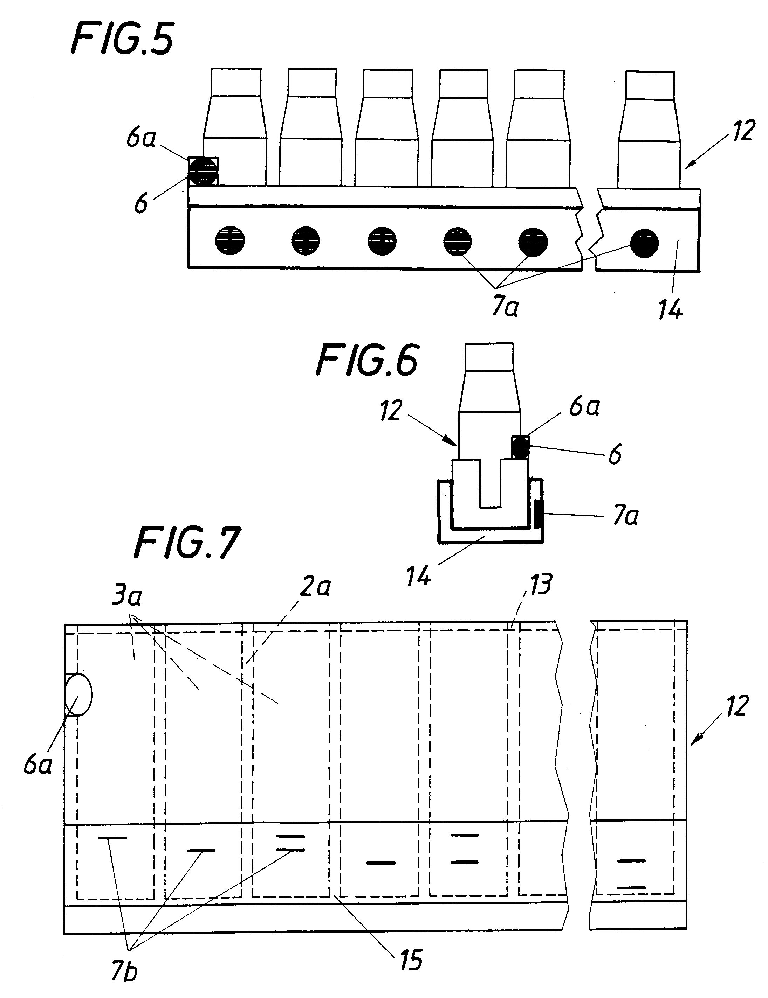

In den Fig. 2 - 4 ist eine Prüfeinheit 12 veranschaulicht, die aus einzelnen, in ein entsprechendes Stativ 2 fest eingesetzten Probenbehältern 3 hergestellt ist. Gemäß Fig. 7 kann die Prüfeinheit 12 aber auch als einstückiger Block 2 a hergestellt sein, in dem von oben zugängliche Hohlräume 3 a als Behälter ausgespart sind, so daß sehr einfache und rationell herstellbare Prüfeinheiten entstehen, wobei selbstverständlich auch hier mit einer gemeinsamen Dekkelleiste 13 ein Einheitsverschluß möglich ist.

Besonders zweckmäßig ist es weiters, wenn die Prüfeinheit 12 mit einem eine Codespur aufweisenden Markierungsstreifen 15 versehen ist, auf den Reihenmarkierungen 7 b zur Bestimmung der jeweiligen Platznummer In der Prüfeinheit aufgebracht sind. Da diese Markierungsstreifen für alle Prüfeinheiten gleichbleiben können, ist der Mehraufwand für diese Codierung nicht sehr groß und es ist möglich, für dip einzelnen Probenbehälter eine individuelle Markierung aus dieser Reihenmarkierung und der jeweiligen Stativmarkierung zu erreichen. Der Markierungsstreifen 15 kann im Zuge der Herstellung der Prüfeinheit 12 aufgebracht werden und führt zu einer wesentlichen Erleichterung sowohl beim Identifizieren der Behälter zum Abfüllen als auch bei der Wiederidentifizierung im Zuge der eigentlichen Milchprüfung.

## Patentansprüche

1. Probenahmevorrichtung für eine Milchqualitätsprüfung mit einem Probenkasten (1) zu Aufnahme mehrerer nebeneinander angeordneter, jeweils eine Reihe von Probenbehältern (3) aufweisender Stative (2), einem Positionsmelder (9) zum Identifizieren der einzelnen Behälter (3) und einer von Behälter zu Behälter relativbewegbaren Abfülleinrichtung (5) zum Einfüllen der Proben in die Behälter, wobei jedes Stativ (2) eine bestimmte Stativmarkierung (6) trägt und in Stativlängsrichtung Reihenmarkierungen (7) für die Behälterpositionen innerhalb einer Behälterreihe vorgesehen sind und der Positionsmelder (9) Leseeinrichtungen (10, 11) zum Auslesen dieser Markierungen (6, 7) in Abhängigkeit von der jeweiligen Lage der Abfülleinrichtung (5) umfaßt, dadurch gekennzeichnet, daß die Stative (2) und die ihnen zugehörenden Probenbehälter (3) jeweils als feste Prüfeinheit (12) ausgebildet sind.

2. Probenahmevorrichtung nach Anspruch 1, dadurch gekennzeichnet, daß die aus Stativ (2a) und Probenbehältern (3a) bestehenden Prüfeinheiten (12) jeweils einstückig hergestellt sind.

3. Probenahmevorrichtung nach Anspruch 1 oder 2, dadurch gekennzeichnet, daß die Prüfeinheiten (12) mit einer sich über die gesamte Behälterreihe erstreckenden Deckelleiste (13) verschließbar sind.

4. Probenahmevorrichtung nach einem der Ansprüche 1-3, dadurch gekennzeichnet, daß die Stativmarkierung (6) auf einem in einem Eckkantenbereich der Prüfeinheit (12) schräg zwischen Stirn- und Längsseite angeordneten Markierungsträger (6a) aufgebracht ist.

5. Probenahmevorrichtung nach einem der Ansprüche 1-3, dadurch gekennzeichnet, daß die Stativmarkierung auf einem an der Unterseite der Prüfeinheit angeordneten Markierungsträger aufgebracht ist.

6. Probenahmevorrichtung nach einem der Ansprüche 1-5, dadurch gekennzeichnet, daß die Prüfeinheiten (12) in eine Markierungsschiene (14) einsetzbar sind, die den Reihenmarkierungen (7) für den Probenkasten (1) entsprechende Positionsmarkierungen (7a) aufweist.

7. Probenahmevorrichtung nach einem der Ansprüche 1-6, dadurch gekennzeichnet, daß die Prüfeinheiten (12) mit einem der Länge nach verlaufenden, die Reihenmarkierungen (7b) tragenden Markierungsstreifen (15) versehen sind.

8. Probenahmevorrichtung nach einem der Ansprüche 1-7, mit einem aus einem in Längsrichtung der Prüfeinheiten (12) verfahrbaren Längsschlitten (4a) und einem in Querrichtung dazu verfahrbaren Querschlitten (4b) zusammengesetzten Kreuzschlitten (4) zur Aufnahme der Abfülleinrichtung (5) und der Leseeinrichtungen (10, 11) des Positionsmelders (9), dadurch gekennzeichnet, daß die Leseeinrichtung (10) für die Stativmarkierungen (6) einen der Lage der jeweiligen Markierungsträger (6a) entsprechend nebeneinandergereihte Einzelsensoren (10b) umfassenden Mehrfachle- sekopf (10a) und einen am Querschlitten (4b) sitzenden, einer längsschlittenfesten Markierungsleiste (10d) zugeordneten Positionsgeber (10e) zur Sensoraktivierung aufweist und daß die Reihenmarkierungen (7) auf einem schlittenunabhängigen Längsträger (8), beispielsweise eine führungsfeste Markierungsleiste, aufgebracht und über einen am Längsschlitten (4a) sitzenden Lesekopf (11a) auslesbar sind.

FIG.1

FIG.2

FIG.3

FIG.4

FIG.5

6a

6

12

7a

14

FIG.6

12

6a

6

7a

14

FIG.7

3a

2a

13

12

6a

7b

15